(19) Europäisches Patentamt
European Patent Office
Office européen des brevets

(11) Veröffentlichungsnummer: **0 044 975**
**A1**

(12) # EUROPÄISCHE PATENTANMELDUNG

(21) Anmeldenummer: **81105208.3**

(22) Anmeldetag: **04.07.81**

(51) Int. Cl.³: **C 08 G 65/32,** C 07 C 69/618, C 08 L 71/02, A 61 K 7/42 // C09D7/12

(30) Priorität: **26.07.80 DE 3028503**

(43) Veröffentlichungstag der Anmeldung: **03.02.82 Patentblatt 82/5**

(84) Benannte Vertragsstaaten: **CH DE FR GB IT LI**

(71) Anmelder: **BASF Aktiengesellschaft,**
**Carl-Bosch-Strasse 38, D-6700 Ludwigshafen (DE)**

(72) Erfinder: **Thoemel, Frank, Dr., Leberstrasse 17, D-6940 Weinheim (DE)**
Erfinder: **Seib, Kail, Dr., Kriemhildstrasse 36, D-6940 Weinheim (DE)**
Erfinder: **Schneider, Kurt, Dr., Auf dem Koeppel, D-6702 Bad Duerkheim (DE)**
Erfinder: **Naegele, Paul, Wiesenstrasse 9, D-6701 Neuhofen (DE)**

(54) **Polyalkoxycarbinolzimtsäureester, Verfahren zu ihrer Herstellung und diese enthaltende Lichtschutzmittel.**

(57) Verbindungen der Formel I

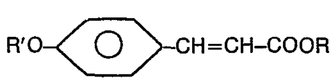

(I),

in der R einen Polyäthylenglykolrest mit 3 bis 60 Kettengliedern oder einen Polypropylenglykolrest mit 3 bis 60 Kettengliedern oder den Rest eines Blockpolymeren aus Äthylenoxid und Propylenoxid mit einem durchschnittlichen Molekulargewicht von 300 bis 8500 und R' eine geradkettige oder verzweigte Alkylgruppe mit 1 bis 10 Kohlenstoffatomen oder einen Benzylrest bedeuten.

Man erhält die Verbindungen in der Weise, daß man einen an der phenolischen Hydroxygruppe entsprechend verätherten p-Hydroxyzimtsäurealkylester mit 1 bis 5 C-Atomen im Alkyl mit einem Polyalkoxycarbinol HOR, in dem R die für Formel I angegebenen Bedeutungen hat, bei Temperaturen von 50 bis 250 °C und in Gegenwart von Alkali umsetzt.

Lichtschutzmittel für die menschliche Haut, enthalten 0,1 bis 15 Gewichtsprozent einer Verbindung der Formel I, neben in der Kosmetik üblichen Trägerstoffen oder Verdünnungsmitteln und gegebenenfalls üblichen kosmetischen Hilfsstoffen.

Die Verbindungen können außerdem als technische Lichtschutzmittel oder UV-Stabilisator Verwendung finden.

EP 0 044 975 A1

Polyalkoxycarbinolzimtsäureester, Verfahren zu ihrer Herstellung und diese enthaltende Lichtschutzmittel

Die vorliegende Erfindung betrifft neue, an der phenolischen Hydroxygruppe verätherte und an der Carbonsäuregruppe polyalkoxylierte Zimtsäureester, ihre Herstellung, diese enthaltende Mittel und ihre Verwendung als Lichtschutzmittel für die menschliche Haut und auf technischem Gebiet als Lichtschutzmittel bzw. UV-Stabilisator.

Es ist bekannt, daß der als UV-B-Strahlung bezeichnete Bereich zwischen 280 und 350 nm des Sonnenlichts oder künstlicher Lichtquellen für die Erythembildung der menschlichen Haut verantwortlich ist. Das Maximum der Wirksamkeit der UV-Strahlung für die Erythembildung liegt bei 297 nm, wenn die Strahlungsintensität für alle Wellenlängen gleich groß ist. Beim Sonnenlicht mit Strahlung unterschiedlicher Intensität ist dieses Maximum auf 308 nm verschoben. Durch geeignete Filtersubstanzen für den UV-B-Bereich gelingt es, die Erythembildung zu verhindern oder zumindest zu verzögern. Die Pigmentbildung der Haut, d.h. die Bräunung, soll dagegen gewährleistet bleiben.

Die UV-Strahlung ist darüber hinaus auch eine wichtige Einflußgröße bei der Alterung von Polymeren und kann beispielsweise die Umwandlung bestimmter Farbstoffe bewirken, so daß auch für solche Produkte Filtersubstanzen als Stabilisatoren nahezu unentbehrlich sind.

In den vergangenen 40 Jahren ist eine große Anzahl von chemischen Verbindungen auf ihre Filterwirkung im UV-B-Bereich hin untersucht worden. Ob es sich jedoch bei einer im

Ze/BL

UV-Gebiet absorbierenden Substanz auch für die menschliche Haut um einen brauchbaren Sonnenschutzfilter handelt, wird noch von anderen Faktoren entscheidend bestimmt:

Neben der hohen Filterwirksamkeit in dem erythemalen Bereich soll die Substanz eine hohe Durchlässigkeit auf die Bräunungsenergie aufweisen, sie sollte eine möglichst ideale Haut- und Schleimhautverträglichkeit besitzen und darf nicht toxisch sein. Sie darf nicht oxidationsempfindlich sein und durch UV-Bestrahlung keine Veränderungen oder Verfärbungen erleiden. Eine entsprechende Zubereitung soll lagerstabil sein, keinen Eigengeruch aufweisen und mit den üblicherweise verwendeten Trägerstoffen oder Verdünnungsmitteln verträglich sein.

Die bekannten UV-Filter weisen häufig als Nachteile auf, daß sie beim Lagern sowie gegen UV-Strahlung oder sichtbare Strahlung und/oder Luft instabil sind, in gefärbte Zersetzungsprodukte übergehen, die Wäsche anschmutzen oder sogar hautschädlich sein können. Da Sonnenschutzmittel viel von Personen verwendet werden, die im Freien arbeiten, Sport treiben, wie Schwimmen und Tauchen, sollen diese Mittel nicht zu leicht durch Wasser oder Schweiß von der Haut entfernbar sein.

In der Praxis haben sich nur verhältnismäßig wenig Substanzen, die den angegebenen Ansprüchen mehr oder weniger gut entsprechen, durchgesetzt, wie beispielsweise der Chemischen Rundschau 24, 1097 ff. (1971) entnommen werden kann. Dazu gehören die p-Methoxyzimtsäureester, welche in physikalischer, toxikologischer und dermatologischer Hinsicht zu den unbedenklichen Lichtschutzmitteln zählen. Weiterhin ist beispielsweise aus der DE-PS 15 43 387

bekannt, daß polyalkoxylierte p-Aminobenzoesäureester als Lichtschutzmittel verwendet werden können. Die Polyalkoxylierung erfolgt dabei durch Umsetzung der entsprechenden Carbonsäuren oder Ester mit Alkylenoxiden in Gegenwart von Alkali. Die Anlagerung der Alkylenoxide kann sowohl an der Carbonsäure- als auch an der Aminogruppe erfolgen.

Es wurde nun gefunden, daß Verbindungen der allgemeinen Formel I

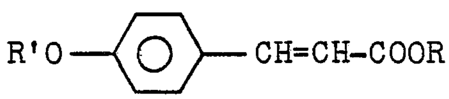 (I)

in der R einen Polyäthylenglykolrest mit 3 bis 60 Kettengliedern oder einen Polypropylenglykolrest mit 3 bis 60 Kettengliedern oder den Rest eines Blockpolymeren aus Äthylenoxid und Propylenoxid mit einem durchschnittlichen Molgewicht von 300 bis 8500 und R' eine geradkettige oder verzweigte Alkylgruppe mit 1 bis 10 Kohlenstoffatomen oder einen Benzylrest bedeuten, in hervorragender Weise als Lichtschutzmittel verwendet werden können.

Von den für R angegebenen Polyäthylenglykol- und Polypropylenglykolresten sind solche mit 5 bis 25 Kettengliedern besonders zu nennen. Die verwendeten Blockpolymeren aus Äthylenoxid und Propylenoxid entsprechen in der Regel dem Typ der allgemeinen Formel II

$$-(CH_2-CH_2-O)_x-(CH(CH_3)-CH_2-O)_y-(CH_2-CH_2-O)_z-H \quad (II),$$

in der x, y, z = 3 sind und bestehen aus 10 bis 80 Gewichtsprozent Polyäthylenoxid und 90 bis 20 Gewichtsprozent Propylenoxid.

Von den für R' genannten Bedeutungen sind bevorzugt die Reste mit 1 bis 4 Kohlenstoffatomen.

Die Verbindungen der Formel I werden hergestellt durch Umsetzung eines an der phenolischen Hydroxygruppe entsprechend verätherten p-Hydroxyzimtsäurealkylesters mit 1 bis 5 Kohlenstoffatomen im Alkyl mit einem Polyalkoxycarbinol HOR, in dem R die für Formel I angegebenen Bedeutungen hat, bei Temperaturen von 50 bis 250°C und in Gegenwart von Alkali.

Diese Umsetzung erfolgt in an sich üblicher Weise. Bevorzugt werden die entsprechenden Zimtsäuremethylester umgesetzt. Als Katalysator für die Umesterungsreaktion verwendet man zweckmäßigerweise Alkalihydroxide, Alkoholate oder Oxide von den Elementen der 1. und 2. Hauptgruppe des Periodensystems, insbesondere des Natriums und Kaliums. Die Umsetzung wird in äquimolarem Verhältnis oder geringem Überschuß an Polyalkoxycarbinol und in Gegenwart von 0,02 bis 1 Moläquivalent, vorzugsweise von 0,05 bis 0,5 Moläquivalent Alkali, bevorzugt bei Temperaturen von 100 bis 200°C und bei Normaldruck oder zweckmäßigerweise unter vermindertem Druck von 0,1 bis 200 mbar durchgeführt.

Die Reaktionszeiten betragen im allgemeinen 15 Minuten bis 20 Stunden. Den Verlauf des Umesterungsprozesses kann man mit Hilfe der Dünnschichtchromatographie (Fertigplatten Kieselgel von Firma Merck, Laufmittel Cyclohexan/Essigsäureäthylester 3 : 1 sowie Methanol, Sprühmittel Kaliumpermanganat in konz. Schwefelsäure) verfolgen.

Den Katalysator gibt man zweckmäßig in wäßriger oder alkoholischer Lösung bei 50 bis 75°C zum Polyalkoxycarbinol und entfernt das Wasser durch Destillation unter Anlegen von vermindertem Druck. Dann gibt man den entsprechenden

Zimtsäureester hinzu, vermindert gegebenenfalls den Druck und erwärmt. Das Alkali kann am Ende des Reaktionsprozesses, nach dem Abkühlen des Gemisches auf 50 bis 20°C, durch Zusatz verdünnter Mineralsäuren unter Rühren neutralisiert werden. Das sich danach im Reaktionsgemisch befindliche Wasser entfernt man wieder durch Destillation unter vermindertem Druck.

Es wird darauf hingewiesen, daß interessanterweise eine direkte Alkoxylierung bei den verätherten p-Hydroxyzimtsäuren nur mäßig oder gar nicht zum Erfolg führt, wobei noch verfärbte Produkte entstehen können.

Die erfindungsgemäßen Verbindungen absorbieren mit sehr guter Extinktion im UV-B-Bereich. Durch Variation der Äther- und Ester-Komponenten können Verbindungen von bestimmter Konsistenz und mit gewünschten Eigenschaften im Hinblick auf die Löslichkeit hergestellt werden. Die Palette der bisher verfügbaren Lichtschutzmittel wird durch die erfindungsgemäß zu verwendenden und chemisch leicht zugänglichen Verbindungen in vorteilhafter Weise bereichert. Ein hervorzuhebender Vorteil der erfindungsgemäßen Verbindungen liegt darin, daß sie relativ gut wasserlöslich sein können. Die Verbindungen können hohe Lichtschutzfaktoren aufweisen (vgl. Ärztliche Kosmetologie, 7, 56 ff (1977)). Die Filterwirkung für den UV-B-Bereich in wäßrigen Lösungen kann allgemein zur Stabilisierung von kosmetischen Zubereitungen, Kunststoffen, Farbstoffen, Lösungs- oder Anstrichmitteln genutzt werden.

Die erfindungsgemäß zu verwendenden Verbindungen können auch in Kombination mit anderen Lichtschutzmitteln Verwendung finden.

Gegenstand der Erfindung ist demnach auch ein als kosmetisches Mittel oder Zubereitung vorliegendes Lichtschutzmittel mit einem Gehalt von 0,1 bis 15 Gewichtsprozent, bevorzugt 2 bis 8 Gewichtsprozent, einer Verbindung der Formel I neben üblichen festen, halbfesten oder flüssigen Trägerstoffen oder Verdünnungsmitteln, Gemischen aus diesen und gegebenenfalls unter Zusatz üblicher kosmetischer Hilfsstoffe.

Von der Art des Trägers oder Verdünnungsmittels hängt es ab, ob das fertige Lichtschutzmittel beispielsweise eine Lösung, ein Öl, eine Creme, eine Salbe, eine Lotion, eine Emulsion oder ein Pulver ist. Derartige Zubereitungen können beispielsweise der Zeitschrift "Fette und Seifen", 53. Jahrgang, Seiten 694-699 (1951), der Zeitschrift "Seifen, Öle, Fette, Wachse", 1955, Seite 147, oder H. Janistyn, Handbuch der Kosmetika und Riechstoffe, 3. Band (1973) entnommen werden.

Üblicherweise verwendete kosmetische Hilfsstoffe, die als Zusätze in Betracht kommen, sind beispielsweise Emulgatoren, wie Fettalkoholäthoxylate, Sorbitanfettsäureester oder Lanolinderivate, Dickungsmittel, wie Carboxymethylcellulose oder vernetzte Polyacrylsäure, Konservierungsmittel oder Parfums.

Grundlage für Sonnenschutzöle sind beispielsweise pflanzliche Öle, wie Erdnußöl, Olivenöl, Sesamöl, Baumwollsamenöl, Kokosöl, Traubenkernöl, Ricinusöl, oder Mineralöle, wie Vaselinöl oder insbesondere flüssiges Paraffin, synthetische Fettsäureester und Glyceride.

Grundlage für Salben sind beispielsweise Vaselin, Lanolin, Eucerin oder Polyäthylenglykole.

Grundlage für Cremes sind beispielsweise fettreiche Cremes, Glycerin-, Polysaccharid-, Tylose-Cremes, für Cremes auf Basis von Fetten und Wachsen Cetylalkohol, Lanolincreme, Kakaobutter, Bienenwachs, Stearinsäure, Stearinalkohol, Glycerinmonostearat, native und mineralische Öle und Fette.

Grundlage für Emulsionen sind beispielsweise Mischungen aus Stearinglykol, einem pflanzlichen und/oder Mineralöl, wie Mandelöl, Paraffinöl und Vaselin und Wasser oder Mischungen aus Äthylalkohol, Wasser, Lanolin und Tragant oder Mischungen aus Äthylalkohol, Stearin, Wasser oder Tragant, Glycerin, Alkohol und Wasser, oder Mischungen aus Stearinsäure, Paraffinöl, Propyl- oder Isopropylalkohol und Wasser.

**Zur Erläuterung des Gegenstands der Erfindung sind nachstehend einige Beispiele angeführt, die aber keinesfalls als einschränkend für die Erfindung anzusehen sind.**

## Beispiel 1

Zu 150 g (136 mmol) Polyäthylenoxid vom mittleren Molekulargewicht 1100 gibt man 1 g (25 mmol) Ätznatron, gelöst in 3 ml Wasser. Man rührt eine Minute bei 75°C gut durch und legt ein Vakuum von 30 mbar an. Nach 5 Minuten setzt man 24 g (125 mmol) p-Methoxyzimtsäuremethylester zu und rührt 4,5 Stunden bei 150°C/30 mbar. Zur Neutralisation des basischen Katalysators setzt man 10 ml 10proz. Schwefelsäure zu, rührt bei 50°C eine Minute gut durch und destilliert bei 50°C/30 mbar Wasser ab. Man erhält 158 g wasserlösliches Reaktionsprodukt; Erstarrungspunkt: 45 bis 55°C; UV-Spektrum (Äthanol) $\lambda_{max}$ 308,8 nm, $\varepsilon = 2,11 \times 10^4$, $E^{1\%}_{1\,cm}$ 3 165.

Beispiel 2

Zu 37,5 g (34 mmol) Polyäthylenoxid vom mittleren Molekulargewicht 1100 gibt man bei 75°C 0,3 g (7,5 mmol) Ätznatron, gelöst in 5 ml Wasser, verrührt eine Minute lang und legt ein Vakuum von 30 mbar an. Nach 5 Minuten setzt man 9 g (31 mmol) p-Octoxyzimtsäuremethylester zu und rührt eine Stunde bei 150°C/30 mbar. Man läßt auf 60°C abkühlen, setzt 5 ml Wasser und 1 ml 10proz. Schwefelsäure zu und destilliert bei 30 mbar Wasser ab. Unter Rühren erhitzt man dabei 10 Minuten auf 60°C und danach kurz bis auf 150°C. Man erhält 43 g Reaktionsprodukt, Erstarrungspunkt: 45 bis 55°C; $\lambda_{max}$ (Äthanol) 309 nm, $\varepsilon = 1,96 \times 10^4$, $E^{1\,\%}_{1\,cm} = 144$.

Beispiel 3

Zu 75 g (68 mmol) Polyäthylenoxid vom mittleren Molekulargewicht 1100 gibt man bei 150°C 0,5 g Ätznatron und setzt nach 2 Minuten 14,7 g (63 mmol) p-tert.-Butoxyzimtsäuremethylester zu. Man erhitzt eine Stunde auf 150°C/30 mbar, läßt das Reaktionsprodukt auf 50°C abkühlen und gibt 6 ml Wasser und 5 ml 10proz. Schwefelsäure zu. Nach 5 Minuten Rühren bei Normaldruck legt man dann ein Vakuum von 30 mbar an und erhitzt im Verlauf von 20 Minuten auf 150°C. Es werden 83 g Reaktionsprodukt erhalten; Erstarrungspunkt: 40 bis 50°C; $\lambda_{max}$ (Äthanol) 292,6 nm, $\varepsilon = 1,88 \times 10^4$, $E^{1\,\%}_{1\,cm} = 144$.

Beispiel 4

Zu 150 g (42,3 mmol) eines Blockpolymeren aus Äthylenoxid und Propylenoxid vom mittleren Molekulargewicht 3750

gibt man bei Raumtemperatur 0,35 g (8,8 mmol) Ätznatron, gelöst in 5 ml Wasser, verrührt einige Minuten, erhitzt dann 5 Minuten lang auf 75°C/30 mbar und setzt danach 7,3 g (38,1 mmol) p-Methoxyzimtsäuremethylester zu. Man erhitzt 3 Stunden auf 150°C/30 mbar und erhält 141 g flüssiges Reaktionsprodukt; $\lambda_{max}$ (Äthanol) 308,5 nm, $\varepsilon$ = 1,9 x $10^4$, $E_{1\,cm}^{1\,\%}$ = 51.

Beispiel 5

Zu 75 g (125 mmol) Polypropylenoxid vom mittleren Molekulargewicht 600 gibt man bei 75°C 1,0 g (15 mmol) Ätznatron, gelöst in 5 ml Wasser, verrührt das Gemisch eine Minute lang und destilliert in 5 Minuten bei 30 mbar Wasser ab. Anschließend gibt man 21,7 g:113 mmol p-Methoxyzimtsäuremethylester zu und erhitzt 6 Stunden bei Normaldruck auf 200°C. Man läßt auf 50°C abkühlen, neutralisiert mit 3,5 ml 10proz. Schwefelsäure analog Beispiel 1 und erhält 81 g flüssiges Reaktionsprodukt; $\lambda_{max}$ (Äthanol) 308,5 nm, $\varepsilon$ = 1,66 x $10^4$, $E_{1\,cm}^{1\,\%}$ = 214.

Beispiel 6

Zur Herstellung einer Sonnenschutzmilch werden

1,7 g Fettalkohol x 6 Ethylenoxid
1,7 g Fettalkohol x 25 Ethylenoxid
4,3 g Äthylhexansäuredecylester
3,9 g Stearinsäuretriglycerid
11,2 g Mineralöl
3,5 g Stearinsäure
0,4 g Cetylalkohol
0,2 g p-Hydroxybenzoesäuremethylester
5,5 g p-Methoxy-2-äthylhexylcinnamat

bei 70°C zusammengeschmolzen. In diese Mischung rührt man ein auf dieselbe Temperatur erwärmtes Gemisch von

1,7 g Triäthanolamin
14,4 g Verbindung gemäß Beispiel 1
0,3 g Parfumöl und
51,2 g Wasser

ein und rührt bis zum Erreichen der Raumtemperatur weiter.

Patentansprüche

1. Verbindungen der Formel I

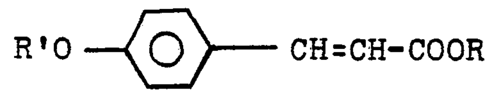 (I) ,

in der R einen Polyäthylenglykolrest mit 3 bis 60 Kettengliedern oder einen Polypropylenglykolrest mit 3 bis 60 Kettengliedern oder den Rest eines Blockpolymeren aus Äthylenoxid und Propylenoxid mit einem durchschnittlichen Molekulargewicht von 300 bis 8500 und R' eine geradkettige oder verzweigte Alkylgruppe mit 1 bis 10 Kohlenstoffatomen oder einen Benzylrest bedeuten.

2. Verfahren zur Herstellung der Verbindungen der Formel I nach Anspruch 1, dadurch gekennzeichnet, daß man einen an der phenolischen Hydroxygruppe entsprechend verätherten p-Hydroxyzimtsäurealkylester mit 1 bis 5 C-Atomen im Alkyl mit einem Polyalkoxycarbinol HOR, in dem R die für die Formel I angegebenen Bedeutungen hat, bei Temperaturen von 50 bis 250°C und in Gegenwart von Alkali umsetzt.

3. Lichtschutzmittel für die menschliche Haut, gekennzeichnet durch einen Gehalt von 0,1 bis 15 Gewichtsprozent einer Verbindung der Formel I, neben in der Kosmetik üblichen Trägerstoffen oder Verdünnungsmitteln und gegebenenfalls üblichen kosmetischen Hilfsstoffen.

4. Verwendung einer Verbindung der allgemeinen Formel I nach Anspruch 1 als Lichtschutzmittel für die menschliche Haut oder als technisches Lichtschutzmittel oder UV-Stabilisator.

Europäisches Patentamt

**EUROPÄISCHER RECHERCHENBERICHT**

0044975

Nummer der Anmeldung

EP 81 10 5208.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A1 - 2 946 831 (STE NATIONALE ELF AQUITAINE) * Ansprüche 5, 6, 11 * | 1 |
| | DE - B2 - 2 146 414 (MITSUBISHI CHEMICAL INDUSTRIES, LTD.) * Anspruch 1; Spalte 2, Zeilen 59 bis 64 * | 1 |
| | CH - A - 306 569 (CIBA AG) * Anspruch * | 2 |
| | US - A - 3 937 810 (G.P. MATHUR et al.) * Anspruch 3 * | 4 |
| P | FR - A - 2 453 132 (SHISEIDO CO. LTD.) * Anspruch 1 * | 4 |
| | DE - A1 - 2 529 511 (HENKEL & CIE GMBH) * Anspruch 4 * | 4 |
| | Chemical Abstracts Band 74, Nr. 24, 14. Juni 1971 Columbus, Ohio, USA F. EIDEN et al. "Analysis of sun protective agents" Seite 319, Spalte 2, Abstract Nr. 130283h & Deut. Apoth. Ztg. Band 111, Nr. 4, 1971, Seiten 118 bis 120 | 4 |

KLASSIFIKATION DER ANMELDUNG (Int. Cl.³)

C 08 G 65/32
C 07 C 69/618
C 08 L 71/02
A 61 K 7/42
// C 09 D 7/12

RECHERCHIERTE SACHGEBIETE (Int. Cl.³)

A 61 K 7/00
C 07 C 57/44
C 07 C 69/618
C 08 G 65/32
C 08 L 71/02
C 09 D 7/12

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 29-09-1981 | IDEZ |